# FASCICULE DE BREVET EUROPEEN

(11) **EP 2 970 115 B1**
(45) Date de publication et mention de la délivrance du brevet: **27.06.2018**
(21) Numéro de dépôt: 14710256.0
(22) Date de dépôt: 14.03.2014
(51) Int. Cl.: C07C 275/60, C08G 18/67, C08G 18/78, C08F 290/06

(54) **ALLOPHANATE POLYACRYLATE**
ALLOPHANATPOLYACRYLAT
ALLOPHANATE POLYACRYLATE

(30) Priorité: 14.03.2013 FR 1352300
(43) Date de publication de la demande: 20.01.2016
(73) Titulaire: Vencorex France, 69800 Saint-Priest (FR)
(72) Inventeur: OLIER, Philippe, F-69007 Lyon (FR)
(74) Mandataire: Dennemeyer & Associates S.A.
(86) Numéro de dépôt international: PCT/EP2014/055052
(87) Numéro de publication internationale: WO 2014/140238

(56) Documents cités:
- WO-A1-2008/145932
- WO-A1-2010/067005
- US-A1- 2010 204 434
- US-A1- 2010 273 938
- US-B2- 6 753 394

## Description

L'invention concerne un composé allophanate modifié et porteur de fonctions acrylates, résultant de la réaction d'un allophanate particulier avec un ester issu de la réaction entre un acide choisi parmi l'acide acrylique et l'acide méthacrylique et au moins un alcool ne comprenant pas de groupement oxyalkylène ou (poly)oxyalkylène.
L'invention concerne également l'utilisation de cet allophanate modifié pour la préparation d'une composition de revêtement hydrophobe réticulable, en particulier par irradiation UV.

On connait des allophanates destinés à des compositions de revêtement. WO-2010/067005 décrit un procédé de préparation d'allophanate ainsi qu'un allophanate et une composition comprenant l'allophanate et destinée à la préparation de compositions de revêtement, en particulier de peinture.

On connaît par ailleurs par US-6 753 394 des uréthane-acrylates réticulables et préparés à partir d'un mélange d'oxyalkyl-polyols et de diisocyanates ou de polyisocyanates et d'ester d'acide acrylique ou méthacrylique.

US-2010/273938 décrit des polyisocyanates comprenant des groupements allophanates et préparés à partir d'isophorone diisocyanate (IPDI). Les résines acrylates polymérisées qui sont divulguées dans ce document ne comprennent pas de fonctions acrylates libres et ne peuvent donc pas réagir sous irradiation UV.

US-2010/204434 décrit la préparation d'allophanates insaturés utilisables pour la préparation de revêtements. Afin de pouvoir maîtriser la viscosité, les compositions durcissables décrites dans ce document requièrent la présence systématique de composés isocyanates modifiés au moyen de caprolactones. Ces compositions ne permettent pas de contrôler le caractère hydrophobe des revêtements préparés.

Toutefois, il existe un besoin d'autres solutions pour la préparation de compositions de revêtement hydrophobes réticulables.
En effet, les compositions de l'état de la technique sont généralement sensibles au vieillissement et à l'oxydation du fait de la présence de groupements oxydes d'éthylène.

Un autre problème fréquemment rencontré avec les compositions de l'état de la technique est qu'elles conduisent à des revêtements sensibles à l'eau ou à l'humidité du fait de la présence de groupements polyéthers. Ces revêtements possèdent généralement un caractère hydrophobe insuffisant.

Un autre problème rencontré avec les compositions de revêtement isocyanate-acrylate de l'état de la technique est lié à leur viscosité trop élevée.
Les solutions de l'état de la technique consistent alors à utiliser un diluant ou un diluant réactif, par exemple sous la forme d'un autre composé à fonctions acrylates ou méthacrylates mais qui sont connus pour présenter des problèmes de toxicité.
Une autre solution connue consiste également à utiliser des polyols comprenant des fonctions oxyalkylènes.
Un autre problème majeur des isocyanate-acrylates de l'état de la technique est lié à la mise en oeuvre lors de leur préparation, de substrats, notamment de monomères isocyanates, qui sont toxiques ou qui présentent des problèmes sanitaires.

Un autre inconvénient des isocyanate-acrylates connus est leur faible rapport entre le nombre de fonctionnalités isocyanates qu'ils contiennent par rapport à leur viscosité. En effet, les isocyanate-acrylates de l'état de la technique qui contiennent un nombre élevé de fonctions uréthanes sont souvent trop visqueux pour être utilisés de manière efficace.

Il existe donc un besoin de compositions à base d'isocyanate-acrylates possédant des propriétés améliorées.
Il existe également un besoin de telles compositions qui permettent de préparer des revêtements ne présentant pas les problèmes des revêtements préparés au moyen des compositions de l'état de la technique.

Ainsi, l'invention fournit un allophanate modifié qui permet d'apporter des solutions à tout ou partie des problèmes des isocyanate-acrylates de l'état de la technique.

L'allophanate modifié selon l'invention est particulièrement avantageux pour sa viscosité réduite tout en évitant la mise en oeuvre de monomères à éviter du fait de leur toxicité. L'allophanate modifié selon l'invention est également particulièrement avantageux pour la préparation de compositions de revêtement dont la résistance aux rayures est améliorée. De plus, l'allophanate modifié selon l'invention permet la préparation de compositions de revêtement dont l'hydrophobie est améliorée. La mesure des angles de contact des revêtements formés est ainsi une caractéristique particulièrement avantageuse obtenue lors de la mise en oeuvre de l'allophanate modifié selon l'invention lors de la préparation de compositions de revêtement.

L'invention concerne un allophanate modifié et préparé ou susceptible d'être préparé selon le procédé comprenant
(a) la préparation d'un allophanate de formule (I) 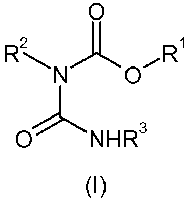 dans laquelle
   ∘ R¹ représente le reste d'un composé monoalcool et comprenant une fonction éther ou polyéther après réaction de l'hydrogène de la fonction OH du monoalcool avec un composé à fonction isocyanate ;
   ∘ R² et R³, identiques ou différents, représentent un groupe hydrocarboné, comprenant une fonction isocyanate dérivée ou non-dérivée ; puis
(b) la réaction avec au moins un ester
   ∘ hydroxy-fonctionnalisé ;
   ∘ comprenant au moins une fonction acrylate et
   ∘ préparé ou susceptible d'être préparé par réaction entre un acide choisi parmi l'acide acrylique et l'acide méthacrylique et au moins un alcool.

Selon la présente invention, une fonction isocyanate non-dérivée est une fonction isocyanate libre pour laquelle le groupement NCO est accessible. Une fonction isocyanate dérivée est une fonction pour laquelle le groupement NCO est lié à un autre groupement chimique.

L'allophanate modifié selon l'invention comprend donc au moins une fonction acrylate. De préférence, il comprend plusieurs fonctions acrylates. Pour cet allophanate modifié selon l'invention, les fonctions acrylates sont liées au reste chimique issu de l'allophanate de formule (I) par l'intermédiaire du reste chimique de l'ester mis en oeuvre. Les fonctions acrylates ne sont donc pas directement liées au reste chimique de l'allophanate de formule (I).

De manière préférée, au sein de l'allophanate modifié selon l'invention, le ratio molaire des fonctions uréthane/allophanate est égal à 2.

Généralement, la fonctionnalité NCO de l'allophanate de formule (I) selon l'invention est égale à 2+/-5%. Cette fonctionnalité peut en effet présenter une légère variabilité autour de la valeur 2, notamment selon les conditions particulières de préparation de cet allophanate de formule (I).
De manière avantageuse, l'allophanate modifié selon l'invention est préparé à partir d'un allophanate de formule (I) qui possède une fonctionnalité NCO choisie parmi une fonctionnalité NCO allant de 1,9 à 2,3 ; une fonctionnalité NCO allant 1,9 à 2,2 ; une fonctionnalité NCO allant de 1,9 à 2,1 ; une fonctionnalité NCO allant de 2 à 2,3 ; une fonctionnalité NCO allant 2 à 2,2.

De manière préférée pour l'allophanate de formule (I), R¹ représente le reste d'un composé monoalcool ne comprenant pas de fonction acrylate.
De manière avantageuse pour l'allophanate de formule (I) selon l'invention, R¹ représente le reste d'un composé monoalcool qui est un composé hydrocarboné comprenant une fonction hydroxyle.
On utilise avantageusement un alcool à chaîne aliphatique incluant les alcools à chaîne cycloaliphatique ou, de préférence un alcool à chaîne alkyle linéaire ou faiblement ramifiée comprenant une seule fonction OH. Il peut s'agir d'un alcool hétérocyclique de type oxétane.
Les alcools appropriés peuvent également éventuellement comprendre une ou plusieurs doubles liaisons.
Le monoalcool utilisé pour la préparation de l'allophanate de formule (I) selon l'invention comprend une fonction éther ou polyéther, avantageusement une fonction (poly)oxyde d'alkylène, de préférence (poly) oxyde d'éthylène, notamment monoéther de polyoxyde d'éthylène comportant avantageusement au plus 25 maillons en moyenne d'oxyde d'éthylène et comportant préférentiellement au plus 10 maillons en moyenne d'oxyde d'éthylène.
D'autres alcools particulièrement avantageux, notamment du point de vue de la faible viscosité, sont les composés de formule R^{a}-[O-CH(R^{b})-CH₂]ₜ-OH, dans laquelle R^{a} représente un groupement C₁-C₂₀-alkyle linéaire ou ramifié ou un groupement de formule R^{c}-CO- dans laquelle R^{c} représente un groupement C₁-C₂₀-alkyle linéaire ou ramifié ; R^{b} représente indépendamment H ou un groupement alkyle, de préférence en C₁-C₈, notamment méthyle, ou un groupement polyéther, notamment un groupement de formule -CH₂OR^{d} dans laquelle R^{d} représente une chaîne hydrocarbonée, notamment polyoxyalkylène, de préférence polyoxyéthylène ; t représente un nombre entier, avantageusement un nombre entier allant de 1 à 10, de préférence de 1 à 5.
Des alcools plus particulièrement avantageux sont les composés de formule R^{a}-[O-CH(R^{b})-CH₂]ₜ-OH, dans laquelle R^{a} représente un groupement C₁-C₂₀-alkyle linéaire ou ramifié ou un groupement de formule R^{c}-CO- dans laquelle R^{c} représente un groupement C₁-C₂₀-alkyle linéaire ou ramifié ; R^{b} représente indépendamment H, un groupement méthyle ou un groupement de formule -CH₂OR^{d} dans laquelle R^{d} représente une chaîne polyoxyéthylène ; t représente un nombre entier allant de 1 à 5.

Comme monoalcools préférés, on peut citer les monoalcools en C₁₂-C₁₈ à 30 fonctions d'oxyde d'éthylène (C₁₂-C₁₈ (30OE)-OH), en particulier issus de mélanges d'alcools en C₁₂-C₁₈, d'alcools en C₁₄-C₁₈ et d'alcools en C₁₆-C₁₈. Ces alcools en C₁₂-C₁₈, en C₁₄-C₁₈ et en C₁₆-C₁₈ peuvent également être utilisés seuls.
Comme exemples particuliers de tels monoalcools, on peut citer les composés choisis parmi le composé CAS RN 68213-23-0, le composé CAS RN 68154-96-1 et le composé CAS RN 68439-49-6 qui peuvent être utilisés seuls ou en mélanges.

La chaîne aliphatique du composé monoalcool peut en outre être substituée ou interrompue par un groupe cycloalkyle ou hétérocyclique dans lesquels la fonction OH peut être liée directement à un atome de carbone de cycle hydrocarboné ou de l'hétérocycle.
Les dérivés de type silanol peuvent également convenir comme composé d'origine du groupement R¹ de l'allophanate de formule (I).
Avantageusement, le monoalcool d'origine du groupement R¹ comprend en moyenne moins de 5 maillons d'oxyde d'alkylène, de préférence en moyenne 2 ou 3 maillons d'oxyde d'alkylène. Il est ainsi possible d'augmenter le titre en NCO de l'allophanate de formule (I).
Lors de la préparation de l'allophanate de formule (I), il peut être avantageux d'utiliser un mélange de composés à fonction(s) alcool(s) différents. Avantageusement, on ne met en oeuvre comme alcool que des monoalcools.
Lors de la préparation de l'allophanate de formule (I), il peut être également avantageux d'utiliser plusieurs monoalcools différents, par exemple au moins 3 monoalcools différents, de préférence au moins 8 monoalcools différents.

Outre les monoalcools, d'autres alcools de type différent peuvent être mis en oeuvre. On cite par exemple les alcools alkyliques à chaîne linéaire en C₁-C₁₀, en particulier les alcools en C₄-C₈.

Pour l'allophanate de formule (I), les groupements R² et R³ peuvent comprendre une fonction isocyanate dérivée ou non-dérivée qui est susceptible de former des fonctions uréthanes par réaction avec un composé porteur d'un atome d'hydrogène labile, en particulier par réaction avec un alcool.

Selon l'invention, pour l'allophanate de formule (I), R² et R³, identiques ou différents, représentent de préférence un groupe comprenant une fonction isocyanate dérivée ou non-dérivée et choisi parmi un groupe hydrocarboné aliphatique, cycloaliphatique, hétérocyclique ou aromatique, de préférence un groupe hydrocarboné aliphatique comprenant une fonction isocyanate dérivée ou non-dérivée.
Pour l'allophanate modifié selon l'invention, l'allophanate de formule (I) est un homo-allophanate et R² et R³ sont identiques ou bien l'allophanate de formule (I) est un allophanate mixte et R² et R³ sont différents.

Selon l'invention, l'allophanate de formule (I) peut être choisi parmi un bis-allophanate, un tris-allophanate, la préparation d'un mélange autres allophanates choisis parmi un ou plusieurs allophanates lourds, ainsi que la préparation de manière minoritaire, du carbamate d'isocyanate R²NCO et d'alcool R¹OH ou du carbamate d'isocyanate R³NCO et d'alcool R¹OH ou du carbamate d'isocyanates R²NCO et R³NCO et d'alcool R¹OH.

De manière préférée, l'allophanate de formule (I) est préparé à partir d'hexaméthylène diisocyanate (HDI) ou d'isophorone diisocyanate (IPDI).

De manière parfois avantageuse, la préparation de l'allophanate modifié selon l'invention peut comprendre la préparation (a) d'un allophanate de formule (I) et également la préparation d'au moins un isocyanate polyfonctionnel.
Comme isocyanate polyfonctionnel, on peut citer les isocyanates polyfonctionnels tricondensats, en particulier les composés de formule (II), ainsi que les isocyanates polyfonctionnels issus de la condensation de plusieurs composés de formule (II) : 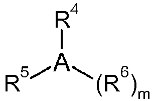 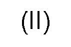 dans laquelle
▪ R⁴, R⁵ et R⁶ représentent indépendamment un groupement hydrocarboné ou hétérocarboné aliphatique, cycloaliphatique, hétérocyclique ou aromatique, comprenant une fonction isocyanate dérivée ou non-dérivée ;
▪ m représente 0, 1 ou 2 ;
▪ A représente un groupement choisi parmi un groupe isocyanurate, un groupe imino oxadiazine dione, un groupe oxadiazine trione, un groupe biuret, respectivement de formules (A1) à (A4) 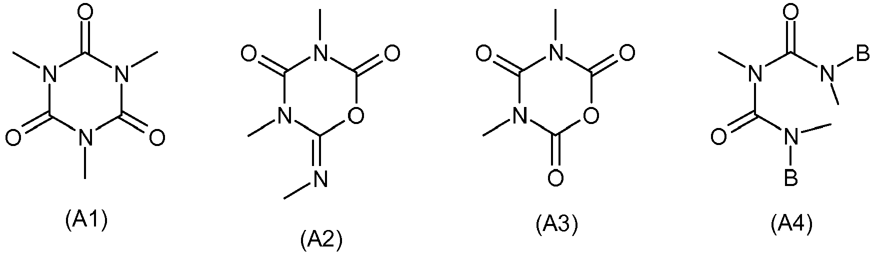 dans laquelle B représente indépendamment un atome d'hydrogène ; un groupe hydrocarboné, notamment un groupe hydrocarboné en C₁-C₂₀; un groupe hétérocarboné comprenant au moins un hétéroatome choisi parmi O, N, S, Si, notamment un groupe hétérocarboné en C₁-C₂₀ comprenant au moins un hétéroatome choisi parmi O, N, S, Si ; un groupement de formule (B1) 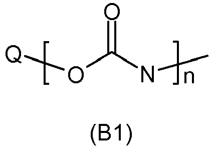 dans laquelle n représente 3 ou 4 et Q représente un groupement choisi parmi un groupe hydrocarboné, un groupement alcoyle, un groupement hydrocarboné, un groupement hétérocarboné aliphatique, cycloaliphatique, hétérocyclique ou aromatique, comprenant une fonction isocyanate dérivée ou non-dérivée.

De manière préférée selon l'invention, l'isocyanate polyfonctionnel tricondensat est un polyisocyanate isocyanurate.

On parlera d'isocyanate polyfonctionnel tricondensat, lorsque R⁴, R⁵, R⁶ identiques ou différents, représentent un groupement de formule -A-X dans laquelle A représente une chaîne hydrocarbonée, c'est-à-dire comportant au moins du carbone et de l'hydrogène et X un atome d'hydrogène ou un groupe NCO, de préférence X représente un groupe NCO. En d'autres termes, par isocyanate polyfonctionnel tricondensat, on entend les produits de (cyclo)condensation théorique obtenus par condensation de trois moles de monomères, avantageusement d'isocyanates, de préférence diisocyanates voire triisocyanates (identiques ou différents), à l'exception des composés provenant de la condensation de plus de quatre monomères ou comportant des groupes allophanates, ainsi que les oligomères isocyanurates obtenus par oligomérisation de (poly)isocyanates isocyanurates.

L'isocyanate polyfonctionnel tricondensat présente avantageusement les caractéristiques :
(i) le rapport pondéral entre l'allophanate de formule (I) et l'isocyanate polyfonctionnel tricondensat va de 60/40 à 90/10, de 30/70 à 90/10, de 60/40 à 80/20, de 30/70 à 80/20, de 60/40 à 85/15 ou de 30/70 à 85/15 ;
(ii) l'isocyanate polyfonctionnel tricondensat est issu d'une réaction de tricondensation pour laquelle le taux de transformation du ou des monomères isocyanates, identiques ou différents, en polyisocyanate polyfonctionnel tricondensat est supérieur à 8% ou supérieur à 10% ou supérieur à 15% ;
(iii) l'isocyanate polyfonctionnel tricondensat comprend entre 1 et 99% en poids de biuret ou entre 2 et 75% en poids de biuret ;
(iv) les combinaisons (i) et (ii), (i) et (iii), (ii) et (iii) ou (i), (ii) et (iii).

La préparation de l'allophanate modifié selon l'invention peut comprendre la préparation (a) d'un allophanate de formule (I) et la préparation d'au moins un isocyanate polyfonctionnel en une proportion inférieure à 10% en masse par rapport à l'allophanate de formule (I) ; en une proportion inférieure à 8% en masse par rapport à l'allophanate de formule (I) ; en une proportion inférieure à 6% en masse par rapport à l'allophanate de formule (I) ; en une proportion inférieure à 2% en masse par rapport à l'allophanate de formule (I).
De manière préférée, l'allophanate modifié selon l'invention est préparé à partir de l'allophanate de formule (I) et en l'absence d'isocyanate polyfonctionnel .

De manière avantageuse, la préparation de l'allophanate modifié comprend (a) la préparation d'un allophanate de formule (I) selon l'invention et la réaction (b) avec au moins un ester réalisée avec un seul ester ou avec deux esters.

Lors de la réaction (b), l'allophanate de formule (I) est déjà formé préalablement et cette réaction (b) ne comprend donc pas d'allophanatation de l'uréthane mis en oeuvre préalablement.
Lors de la mise en oeuvre de la réaction (b), le catalyseur d'allophanatation n'est donc en général plus présent dans le milieu réactionnel sous une forme active. Le catalyseur d'allophanatation est généralement neutralisé.

De manière avantageuse, l'ester mis en oeuvre lors de la réaction (b) du procédé de préparation de l'allophanate modifié selon l'invention est préparé à partir d'un alcool ne comprenant pas de groupement oxyalkylène ou (poly)oxyalkylène, en particulier à partir d'un alcool ne comprenant pas de groupement oxytéthylène ou (poly)oxyéthylène.
Cet ester peut être préparé à partir d'un alcool choisi parmi les composés de formule (III) 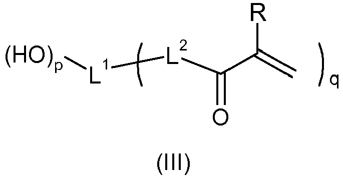 dans laquelle
▪ p représente 1, 2, 3, 4 ou 5 ;
▪ L¹ représente un radical hydrocarboné, linéaire ou ramifié, ou un radical, linéaire ou ramifié, comprenant une chaîne hydrocarbonée et au moins un hétéroatome ;
▪ L² représente O, S ou un groupement de formule NT dans laquelle T représente H ou un groupement C₁-C₈-alkyl linéaire ou ramifié et N représente un atome d'azote ;
▪ R, identique ou différent, représente H ou un groupement C₁-C₈-alkyl linéaire ou ramifié ;
▪ q représente 1, 2, 3, 4 ou 5.
De manière préférée, l'alcool peut être choisi parmi les composés de formule (III) dans laquelle p représente 1 ou 2, en particulier 1 ; q représente 3 ; L¹ représente un radical, linéaire ou ramifié, comprenant une ou plusieurs fonctions éthers ou un composé de formule (III) combinant ces caractéristiques.
L'ester peut également être formé à partir d'un mélange de composés dont la formule moyenne est un composé de formule (III).

Comme exemple d'alcool mis en oeuvre lors de la préparation de l'ester de la réaction (b) selon l'invention, on peut citer le trimethyl-ol-propane (TMP).

De manière préférée, la réaction (b) est mise en oeuvre avec au moins un ester qui est monohydroxy-fonctionnalisé.

De manière également préférée, la réaction (b) est mise en oeuvre avec au moins un ester choisi parmi les 2-hydoxyalkyl(meth)acrylates, le 2-hydroxyethyl(meth)acrylate, le 2-hydroxypropyl(meth)acrylate, le 3-hydroxypropyl(meth)acrylate, le 4-hydroxybutyl(meth)acrylate, le 3-hydroxy-2,2-dimethylpropyl(meth)acrylate, le 2-hydroxyethyl acrylate, le 2-hydroxypropyl acrylate, une caprolactone modifiée par estérification avec des hydroxyalkyl(meth)acrylates, une ε-caprolactone modifiée par estérification avec des hydroxyalkyl(meth)acrylates, une ε-caprolactone modifiée par estérification avec des hydroxyalkylacrylates, une ε-caprolactone modifiée par estérification avec des 2-hydroxyalkyl(meth)acrylates, une ε-caprolactone modifiée par estérification avec des 2-hydroxyalkylacrylates, le caprolactone triacrylate, le glycerol di(meth)acrylate, le trimethylolpropane di(meth)acrylate, le pentaerythritol tri(meth)acrylate, le di(pentaerythritol) penta(meth)acrylate, trimethyl-ol-propane-diacrylate.
De manière plus préférée, la réaction (b) est mise en oeuvre avec au moins un ester choisi parmi les 2-hydoxyalkyl(meth)acrylates, le 2-hydroxyethyl(meth)acrylate, le 2-hydroxypropyl(meth)acrylate, le 3-hydroxypropyl(meth)acrylate, le 4-hydroxybutyl(meth)acrylate, le 3-hydroxy-2,2-dimethylpropyl(meth)acrylate, le 2-hydroxyethyl acrylate, le 2-hydroxypropyl acrylate, le glycerol di(meth)acrylate, le trimethylolpropane di(meth)acrylate, le pentaerythritol tri(meth)acrylate, le di(pentaerythritol) penta(meth)acrylate, trimethyl-ol-propane-diacrylate.
De préférence, l'ester est choisi parmi le pentaerythritol triacrylate, le dipentaerythritol pentaacrylate (DPHA) et le trimethyl-ol-propane-diacrylate.

De manière particulièrement avantageuse, l'allophanate modifié selon l'invention possède une viscosité mesurée à 25°C comprise dans les gammes de viscosité suivantes : de 500 à 200 000 mPa.s ; de 500 à 150 000 mPa.s ; de 500 à 120 000 mPa.s ; de 500 à 100 000 mPa.s; de 500 à 30 000 mPa.s ; de 500 à 50 000 mPa.s ; de 500 à 15 000 mPa.s ; de 1 000 à 200 000 mPa.s ; de 1 000 à 150 000 mPa.s ; de 1 000 à 120 000 mPa.s ; de 1 000 à 100 000 mPa.s ; de 1 000 à 30 000 mPa.s ; de 1 000 à 15 000 mPa.s ; de 2 000 à 100 000 mPa.s ; de 2 000 à 50 000 mPa.s ; de 2 000 à 30 000 mPa.s ; de 3 000 à 30 000 mPa.s ; de 3 000 à 15 000 mPa.s.

L'invention concerne également l'utilisation d'un allophanate modifié selon l'invention pour la préparation d'une composition de revêtement, en particulier d'une composition de revêtement réticulable, notamment d'une composition de revêtement réticulable par irradiation UV.

L'invention concerne également l'utilisation d'un allophanate modifié selon l'invention pour la préparation d'une composition de revêtement hydrophobe, en particulier d'une composition de revêtement hydrophobe réticulable, notamment d'une composition de revêtement hydrophobe réticulable par irradiation UV.

Les caractéristiques particulières, avantageuses ou préférées de l'allophanate modifié selon l'invention constituent des caractéristiques particulières, avantageuses ou préférées de la préparation d'une composition de revêtement, notamment d'une composition de revêtement hydrophobe. Il en est de même pour la préparation d'une composition de revêtement hydrophobe réticulable, notamment réticulable par irradiation UV.

Les différents aspects et les propriétés avantageuses de l'invention peuvent être illustrés par les exemples qui suivent. Ces exemples ne constituent pas de limitation de la portée de cette invention.

### Exemples

Les produits suivants sont utilisés.
HDI : hexaméthyldiisocyanate - société Vencorex
Tolonate™ HDB : biuret de l'hexamethylenediisocyanate - société Vencorex
Poids équivalent NCO = 191 g
Viscosité = 9 000 mPa.s
Extrait sec = 100 %
Tolonate™ IDT 70 B : trimère de l'isophorone diisocyanate - société Vencorex
Poids équivalent NCO = 342 g
Viscosité = 600 mPa.s
Extrait sec = 70 %
(Pentaerythritol) tri-acrylate : produit de réaction de l'acide acrylique et du pentaérythrytol Teneur en groupes OH = 149 mg KOH/g.
Hexanediol diacrylate (HDDA) : diluant réactif acrylé - société Sartomer
Alcool éthoxylé en C₁₂-C₁₈ de teneur en OH égale à 170 mg KOH/g (RNCAS = 68213-23-0)
Catalyseur K KAT XK-629 : solution à 20% dans le 2-ethylhexanol de tris(2-ethylhexanoate de bismuth)

### Exemple 1 : préparation d'un allophanate de formule (I)

Dans un réacteur double-enveloppe parfaitement agité, nous introduisons : 425 g de HDI (2,53 mol) puis 82 g d'un alcool ethoxylé en C₁₂-C₁₈ (0,23 mol) préchauffé à 40°C + 1,45 g de mélange butanol-1 / butanol-2 (75/25 comme rapport massique m/m) et 5,85 g de catalyseur K KAT XK-629 à température ambiante. Le milieu est chauffé pour atteindre la température de 110°C en 2 heures. Le milieu réactionnel est maintenu à cette température pendant 1,5 heure environ.
Le titre NCO du milieu réactionnel est régulièrement mesuré par méthode de dosage retour à la dibutylamine.
La réaction est stoppée par ajout de 0,066 g d'acide para-toluène sulfonique quand le titre NCO du milieu réactionnel correspond au titre théorique attendu.
Après 15 minutes, la température du milieu réactionnel est ramenée à température ambiante.
Le titre NCO du milieu réactionnel final est de 0,829 mol de NCO pour 100 g.
On procède ensuite à 2 distillations successives sur évaporateur film mince sous vide (0,5 mbar environ) à la température de 130°C pour éliminer la plus grande partie du monomère n'ayant pas réagi.
La quantité obtenue après distillation est de 192 g. Ce qui correspond à un rendement de l'ordre de 40 %.

L'allophanate de formule (I) final est caractérisé par les données suivantes :
- titre NCO : 0,30 mol de NCO pour 100 g, soit un pourcentage pondéral de 12,6 % ;
- viscosité mesurée à 25°C : 138 mPa.s.

### Exemple 2 : préparation d'un allophanate modifié selon l'invention

Dans un ballon tricol équipé d'un système de refroidissement, d'un agitateur mécanique et d'une entrée d'azote, on introduit 80 g (0,205 mol) de (pentaerythritol) tri-acrylate (PETIA), 0,02 g de dilaurate de dibutyletain (DBTL), 0,072 g de butylhydroxytoluene (BHT) et 100 g de toluène séché.

On ajoute ensuite sous agitation et goutte à goutte 61,4 g (0,185 mol) de l'allophanate de formule (I) de l'exemple 1 puis le milieu réactionnel est chauffé jusqu'à une température de 60°C.
La réaction est stoppée au bout de 7 h lorsque les groupes NCO ont entièrement réagi et on laisse le milieu réactionnel revenir à température ambiante.
Le solvant est ensuite évaporé sous vide.

### Exemples comparatifs 3 à 5

La même procédure de synthèse est répétée en utilisant à titre de comparaison les polyisocyanates selon le tableau 1.

**Tableau 1**

| Exemple comparatif | Isocyanate de départ | isocyanate (g) | PETIA (g) | toluène (g) | DBTL (g) | Durée de réaction |
|---|---|---|---|---|---|---|
| 3 | Tolonate IDT 70 B | 63 | 80 | 100 | 0,02 | >24 h |
| 4 | Tolonate HDB | 35,2 | 80 | 1400 | 0,02 | 7H30 |
| 5 | HDI | 14,2 | 80,3 | 80 | 0.02 | 7H |

Les caractéristiques des produits obtenus sont présentées dans le tableau 2.

**Tableau 2**

| Exemple | Isocyanate de départ | Teneur en groupes OH (mg KOH/g) | Extrait sec (%) | Mn (g/mol) Mw (g/mol) | Viscosité (Pa.s) |
|---|---|---|---|---|---|
| 2 | Allophanate de l'exemple 1 | 41 | 97,6 | 1100 | 17,1 à 23°C |
| | | | | 1200 | |
| 3 | Tolonate IDT 70 B | 13 | 56,1 | 1300 | 20 à 50°C |
| | | | | 3100 | |
| 4 | Tolonate HDB | 17 | 63,9 | 1400 | 78 à 50°C |
| | | | | 5800 | |
| 5 | HDI | 56 | 97,9 | 800 | 24,3 à 23°C |
| | | | | 1100 | |

L'utilisation de l'allophanate de l'exemple 1 comme réactif de départ permet d'obtenir des produits de viscosité plus faible à la fois en comparaison avec des isocyanates (HDI) et avec des polyisocyanates connus comme les produits Tolonate IDT 70 B ou Tolonate HDB (tableau 2).

### Exemples 6 à 8 : réalisation de revêtements à partir des produits des exemples 2, 3, 4

Les produits des exemples 2, 3 et 4 ont été utilisés pour réaliser un revêtement réticulable sous UV dans les conditions présentées dans le tableau 3.

Les formulations à base d'uréthanes acrylates sont ajustées à 50% d'extrait sec avec de l'acétone puis on ajoute 4 % d'un photoinitiateur (Irgacure 500).
L'application est réalisée sur des plaques de polycarbonate avec une barre K de 12 µm. Après évaporation des solvants (30 minutes à 60°C dans un four), les plaques sont stockées 24 h en conditions de température et d'humidité constante (50% HR, 23°C). L'épaisseur du revêtement est alors de 6 µm.
Les plaques sont ensuite réticulées sous UV (lampe mercure) dans les conditions présentées dans le tableau 3.

**Tableau 3**

| Vitesse défilement | Dose UV-C J/cm² | Dose UV-B J/cm² | Dose UV-A J/cm² | Dose UV-V J/cm² |
|---|---|---|---|---|
| 3 x 5 m/min | 0,213 | 1,41 | 2,148 | 1,917 |

L'évaluation des propriétés suivantes est réalisée 24 h après la réticulation.

### Brillance

Elle est mesurée selon un angle de 20° initialement et après 50 allers-retours avec de la laine de verre lestée d'un poids de 385 g de façon à évaluer un endommagement de la surface due au frottement.

### Anale de contact

La mesure de l'angle formé par une goutte d'eau au contact du revêtement est une indication de l'hydrophobie du revêtement. Plus l'angle est élevé, plus la surface est hydrophobe.

### Dureté crayon

Le revêtement est rayé avec des mines de graphite de dureté croissante selon l'échelle de la figure 1.

**Tableau 4**

| Exemple | Produit (isocyanate de départ) | Dureté crayon | Angle de contact avec l'eau | Brillant (20°) initial | Brillant (20°) après 50 rub |
|---|---|---|---|---|---|
| 6 | Exemple 2 (Allophanate de l'exemple 1) | 5H-6H | 73 | 90 | 88 |
| 7 | Exemple 3 (Tolonate IDT 70 B) | 3H-4H | 65 | 90 | 86 |
| 8 | Exemple 4 (Tolonate HDB) | 3H-4H | 50 | 76 | 89 |

La dureté retenue est celle pour laquelle il n'y a pas de marque sur le revêtement.
Les résultats obtenus montrent que la mise en oeuvre de l'allophanate de formule (I) selon l'exemple 1 lors de la préparation et la mise en oeuvre d'un allophanate modifié selon l'invention permet d'obtenir des revêtements hydrophobes et résistants à la rayure.

### Exemples 9 à 10 : réalisation de revêtements à partir des produits des exemples 2 et 5

Les produits des exemples 2 et 5 sont formulés selon le tableau 5.

**Tableau 5**

| Produit (isocyanate de départ) | Exemple 9 | Exemple 10 |
|---|---|---|
| Exemple 5 (HDI) | 10 | - |
| Exemple 2 (Allophanate de l'exemple 1) | - | 10 |
| Hexanediol diacrylate (HDDA) | 2,5 | 2,5 |
| Irgacure 500 | 0,25 | 0,25 |

Ces formulations sont ensuite appliquées sur des plaques de verre ou d'acier selon le test à l'aide d'une barre K. L'épaisseur sèche est de 35 µm.
L'irradiation sous UV est réalisée à l'aide d'une lampe mercure selon les conditions du tableau 6.

**Tableau 6**

| Vitesse défilement | Dose UV-totale |
|---|---|
| 2 x 5 m/min | 1234 mW/cm² |

La formulation comprenant le produit de l'exemple 2 (issu de l'allophanate de l'exemple 1) possède les mêmes caractéristiques de dureté et d'aspect que l'exemple comparatif mais présente une flexibilité supérieure lors du test embouti Erichsen (ISO 1520-1999) (tableau 7).

**Tableau 7**

| | Exemple 9 | Exemple 10 |
|---|---|---|
| Produit | Exemple 5 (HDI) | Exemple 2 (Allophanate de l'exemple 1) |
| Flexibilité - embouti Erichsen (mm) | 0,3 | 1,4 |
| Brillance (20°C) | 82 | 85 |
| Résistance MEK (double rubs) | >250 | >250 |
| Dureté crayon | 9H | 9H |

## Revendications

1. Allophanate modifié et préparé ou susceptible d'être préparé selon un procédé comprenant
(a) la préparation d'un allophanate de formule (I) 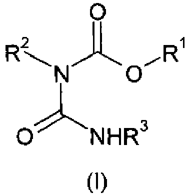 dans laquelle
∘ R¹ représente le reste d'un composé monoalcool et comprenant une fonction éther ou polyéther après réaction de l'hydrogène de la fonction OH du monoalcool avec un composé à fonction isocyanate ;
∘ R² et R³, identiques ou différents, représentent un groupe hydrocarboné, comprenant une fonction isocyanate dérivée ou non-dérivée ;
puis
(b) la réaction avec au moins un ester
∘ hydroxy-fonctionnalisé ;
∘ comprenant au moins une fonction acrylate et
∘ préparé ou susceptible d'être préparé par réaction entre un acide choisi parmi l'acide acrylique et l'acide méthacrylique et au moins un alcool.

2. Allophanate modifié selon la revendication 1 dont le ratio molaire des fonctions uréthane/allophanate est égal à 2.

3. Allophanate modifié selon les revendications 1 ou 2 pour lequel l'allophanate de formule (I) possède une fonctionnalité NCO choisie parmi une fonctionnalité NCO allant de 1,9 à 2,3 ; une fonctionnalité NCO allant 1,9 à 2,2 ; une fonctionnalité NCO allant de 1,9 à 2,1 ; une fonctionnalité NCO allant de 2 à 2,3 ; une fonctionnalité NCO allant 2 à 2,2 ; ou
▪ pour lequel R¹ représente le reste d'un composé monoalcool ne comprenant pas de fonction acrylate, de préférence R¹ représente le reste d'un alcool de formule R^{a}-[O-CH(R^{b})-CH₂]ₜ-OH, dans laquelle R^{a} représente un groupement C₁-C₂₀-alkyle linéaire ou ramifié ou un groupement de formule R^{c}-CO- dans laquelle R^{c} représente un groupement C₁-C₂₀-alkyle linéaire ou ramifié ; R^{b} représente H ou un groupement alkyle, ou un groupement polyéther ; t représente un nombre entier, de préférence t représente un nombre entier allant de 1 à 10 ou R^{b} représente un groupement alkyle en C₁-C₈ ou un groupement polyéther ou bien t représente un nombre entier allant 1 à 5 ou R^{b} représente un méthyle ou un groupement polyéther de formule -CH₂OR^{d} dans laquelle R^{d} représente une chaîne hydrocarbonée, ou bien R^{d} représente une chaîne polyoxyalkylène, une chaîne polyoxyéthylène ; ou
▪ pour lequel R² et R³, identiques ou différents, représentent un groupe comprenant une fonction isocyanate dérivée ou non-dérivée et choisi parmi un groupe hydrocarboné aliphatique, cycloaliphatique, hétérocyclique ou aromatique, de préférence R² et R³, identiques ou différents, représentent un groupe comprenant une fonction isocyanate dérivée ou non-dérivée et choisi parmi un groupe hydrocarboné aliphatique comprenant une fonction isocyanate dérivée ou non-dérivée.

4. Allophanate modifié selon les revendications 1 à 3 pour lequel l'allophanate de formule (I) est un homo-allophanate, R² et R³ étant identiques, ou pour lequel l'allophanate de formule (I) est un allophanate mixte, R² et R³ étant différents.

5. Allophanate modifié selon les revendications 1 à 4 comprenant la préparation d'un allophanate de formule (I) choisi parmi un bis-allophanate, un tris-allophanate, la préparation d'un mélange d'autres allophanates choisis parmi un ou plusieurs allophanates lourds, ainsi que la préparation de manière minoritaire, du carbamate d'isocyanate R²NCO et d'alcool R¹OH ou du carbamate d'isocyanate R³NCO et d'alcool R¹OH ou du carbamate d'isocyanates R²NCO et R³NCO et d'alcool R¹OH.

6. Allophanate modifié selon les revendications 1 à 5 pour lequel l'allophanate de formule (I) est préparé à partir d'hexaméthylène diisocyanate (HDI) ou d'isophorone diisocyanate (IPDI).

7. Allophanate modifié selon les revendications 1 à 6 comprenant la préparation (a) d'un allophanate de formule (I) et la préparation d'au moins un isocyanate polyfonctionnel.

8. Allophanate modifié selon les revendications 1 à 7 comprenant la préparation (a) d'un allophanate de formule (I) et la préparation d'au moins un isocyanate polyfonctionnel tricondensat.

9. Allophanate modifié selon les revendications 1 à 8 comprenant la préparation (a) d'un allophanate de formule (I) et la préparation d'au moins un isocyanate polyfonctionnel, notamment d'au moins un isocyanate polyfonctionnel tricondensat, en une proportion inférieure à 10% en masse par rapport à l'allophanate de formule (I) ; en une proportion inférieure à 8% en masse par rapport à l'allophanate de formule (I) ; en une proportion inférieure à 6% en masse par rapport à l'allophanate de formule (I) ; en une proportion inférieure à 2% en masse par rapport à l'allophanate de formule (I).

10. Allophanate modifié selon les revendications 1 à 9 pour lequel la réaction (b) est réalisée avec au moins un ester monohydroxy-fonctionnalisé.

11. Allophanate modifié selon les revendications 1 à 10 pour lequel la réaction (b) est réalisée avec un seul ester ou avec deux esters ; ou
pour lequel la réaction (b) est réalisée avec au moins un alcool ne comprenant pas de groupement oxyalkylène ou (poly)oxyalkylène.

12. Allophanate modifié selon les revendications 1 à 11 pour lequel la réaction (b) met en oeuvre un ester préparé à partir d'un alcool choisi parmi les composés de formule (III) 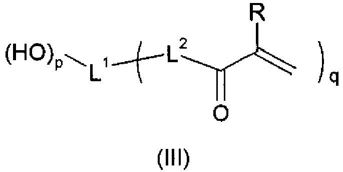 dans laquelle
▪ p représente 1, 2, 3, 4 ou 5 ;
▪ L¹ représente un radical hydrocarboné, linéaire ou ramifié, ou un radical, linéaire ou ramifié, comprenant une chaîne hydrocarbonée et au moins un hétéroatome ;
▪ L² représente O, S ou un groupement de formule NT dans laquelle T représente H ou un groupement C₁-C₈-alkyl linéaire ou ramifié et N représente un atome d'azote ;
▪ R, identique ou différent, représente H ou un groupement C₁-C₈-alkyl linéaire ou ramifié ;
▪ q représente 1, 2, 3, 4 ou 5.

13. Allophanate modifié selon les revendications 1 à 12 pour lequel la réaction (b) est réalisée avec au moins un ester choisi parmi les 2-hydoxyalkyl(meth)acrylates, le 2-hydroxyethyl(meth)acrylate, le 2-hydroxypropyl(meth)acrylate, le 3-hydroxypropyl(meth)acrylate, le 4-hydroxybutyl(meth)acrylate, le 3-hydroxy-2,2-dimethylpropyl(meth)acrylate, le 2-hydroxyethyl acrylate, le 2-hydroxypropyl acrylate, une caprolactone modifiée par estérification avec des hydroxyalkyl(meth)acrylates, une ε-caprolactone modifiée par estérification avec des hydroxyalkyl(meth)acrylates, une ε-caprolactone modifiée par estérification avec des hydroxyalkylacrylates, une ε-caprolactone modifiée par estérification avec des 2-hydroxyalkyl(meth)acrylates, une ε-caprolactone modifiée par estérification avec des 2-hydroxyalkylacrylates, le caprolactone triacrylate, le glycerol di(meth)acrylate, le trimethylolpropane di(meth)acrylate, le pentaerythritol tri(meth)acrylate (PETIA), le di(pentaerythritol) penta(meth)acrylate, le trimethyl-ol-propane-diacrylate.

14. Allophanate modifié selon les revendications 1 à 13 pour lequel la réaction (b) est réalisée avec au moins un ester choisi parmi le pentaerythritol triacrylate (PETIA), le dipentaerythritol pentaacrylate (DPHA), le trimethyl-ol-propane-diacrylate.

15. Utilisation d'un allophanate modifié selon les revendications 1 à 14 pour la préparation d'une composition de revêtement ; pour la préparation d'une composition de revêtement réticulable ; pour la préparation d'une composition de revêtement réticulable par irradiation UV, de préférence pour la préparation d'une composition de revêtement hydrophobe ; pour la préparation d'une composition de revêtement hydrophobe réticulable ; pour la préparation d'une composition de revêtement hydrophobe réticulable par irradiation UV.

## Patentansprüche

1. Allophanat, das nach einem Verfahren modifiziert und hergestellt wird oder hergestellt werden kann, das Folgendes umfasst
(a) die Herstellung eines Allophanats der Formel (I) 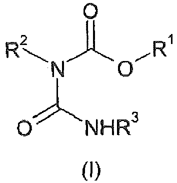 wobei:
∘ R¹ für den Rest einer Monoalkoholverbindung steht und nach Reaktion des Wasserstoffs der OH-Funktion des Monoalkohols mit einer Verbindung mit Isocyanat-Funktion eine Ether- oder Polyether-Funktion umfasst;
∘ R² und R³ identisch oder verschieden für eine Kohlenwasserstoffgruppe stehen, die eine derivatisierte oder nicht derivatisierte Isocyanat-Funktion umfassen;
anschließend
(b) die Reaktion mit mindestens einem Ester, der
∘ hydroxyfunktionalisiert ist,
∘ mindestens eine Acrylat-Funktion umfasst und
∘ durch eine Reaktion zwischen einer Säure, ausgewählt aus Acrylsäure und der Methacrylsäure, und mindestens einem Alkohol hergestellt wird oder hergestellt werden kann.

2. Modifiziertes Allophanat nach Anspruch 1, bei dem das Molverhältnis der Funktionen Urethan/Allophanat gleich 2 ist.

3. Modifiziertes Allophanat nach Anspruch 1 oder 2, wobei das Allophanat der Formel (I) eine NCO-Funktionalität besitzt, ausgewählt aus einer NCO-Funktionalität von 1,9 bis 2,3; einer NCO-Funktionalität von 1,9 bis 2,2; einer NCO-Funktionalität von 1,9 bis 2,1; einer NCO-Funktionalität von 2 bis 2,3 und einer NCO-Funktionalität von 2 bis 2,2 ;oder
▪ wobei R¹ für den Rest einer Monoalkoholverbindung steht, der keine Acrylat-Funktion umfasst, R¹ vorzugsweise für den Rest eines Alkohols der Formel R^{a}-[O-CH(R^{b})-CH₂]ₜ-OH steht, wobei R^{a} für eine lineare oder verzweigte C₁-C₂₀-Alkylgruppe oder eine Gruppe der Formel R^{c}-CO- steht, in welcher R^{c} für eine lineare oder verzweigte C₁-C₂₀-Alkylgruppe steht; R^{b} für H oder eine Alkylgruppe oder eine Polyethergruppe steht; t für eine ganze Zahl steht, t vorzugsweise für eine ganze Zahl zwischen 1 und 10 steht oder R^{b} für eine C-₁-C₈-Alkylgruppe oder eine Polyethergruppe steht oder t für eine ganze Zahl zwischen 1 und 5 steht oder R^{b} für ein Methyl oder eine Polyethergruppe der Formel -CH₂OR^{d} steht, in welcher R^{d} für eine Kohlenwasserstoffkette steht, oder R^{d} für eine Polyoxyalkylenkette oder eine Polyoxyethylenkette steht, oder
▪ wobei R² und R³ identisch oder verschieden für eine Gruppe stehen, die eine derivatisierte oder nicht derivatisierte Isocyanat-Funktion umfasst und ausgewählt aus einer aliphatischen, cycloaliphatischen, heterocyclischen oder aromatischen Kohlenwasserstoffgruppe, wobei R² und R³ identisch oder verschieden vorzugsweise für eine Gruppe stehen, die eine derivatisierte oder nicht derivatisierte Isocyanat-Funktion umfasst und ausgewählt aus einer aliphatischen Kohlenwasserstoffgruppe, die eine derivatisierte oder nicht derivatisierte Isocyanat-Funktion umfasst.

4. Modifiziertes Allophanat nach Anspruch 1 bis 3, wobei das Allophanat der Formel (I) ein Homo-Allophanat ist, wobei R² und R³ identisch sind, oder wobei das Allophanat der Formel (I) ein gemischtes Allophanat ist, wobei R² und R³ verschieden sind.

5. Modifiziertes Allophanat nach Anspruch 1 bis 4, umfassend die Herstellung eines Allophanats der Formel (I), ausgewählt aus einem Bis-Allophanat oder einem Tris-Allophanat, die Herstellung einer Mischung anderer Allophanate, ausgewählt aus einem oder mehreren schweren Allophanaten, sowie die weniger bedeutende Herstellung des Isocyanatcarbamats R²NCO und des Alkohols R¹OH oder des Isocyanatcarbamats R³NCO und des Alkohols R¹OH oder der Isocyanatcarbamate R²NCO und R³NCO und des Alkohols R¹OH.

6. Modifiziertes Allophanat nach Anspruch 1 bis 5, wobei das Allophanat der Formel (I) ausgehend von Hexamethylendiisocyanat (HDI) oder Isophorondiisocyanat (IPDI) hergestellt wird.

7. Modifiziertes Allophanat nach Anspruch 1 bis 6, umfassend die Herstellung (a) eines Allophanats der Formel (I) und die Herstellung von mindestens einem polyfunktionellen Isocyanat.

8. Modifiziertes Allophanat nach Anspruch 1 bis 7, umfassend die Herstellung (a) eines Allophanats der Formel (I) und die Herstellung von mindestens einem polyfunktionellen Isocyanat-Trikondensat.

9. Modifiziertes Allophanat nach Anspruch 1 bis 8, umfassend die Herstellung (a) eines Allophanats der Formel (I) und die Herstellung mindestens eines polyfunktionellen Isocyanats, insbesondere mindestens eines polyfunktionellen Isocyanat-Trikondensats, mit einem Anteil von weniger als 10 Massen-% bezogen auf das Allophanat der Formel (I); einem Anteil von weniger als 8 Massen-% bezogen auf das Allophanat der Formel (I); einem Anteil von weniger als 6 Massen-% bezogen auf das Allophanat der Formel (I); einem Anteil von weniger als 2 Massen-% bezogen auf das Allophanat der Formel (I).

10. Modifiziertes Allophanat nach Anspruch1 bis 9, wobei die Reaktion (b) mit mindestens einem monohydroxyfunktionalisierten Ester durchgeführt wird.

11. Modifiziertes Allophanat nach Anspruch 1 bis 10, wobei die Reaktion (b) mit nur einem einzigen Ester oder mit zwei Estern durchgeführt wird; oder
wobei die Reaktion (b) mit mindestens einem Alkohol durchgeführt wird, der keine Oxyalkylen- oder (Poly)oxyalkylengruppe umfasst.

12. Modifiziertes Allophanat nach Anspruch 1 bis 11, wobei die Reaktion (b) einen Ester einsetzt, der ausgehend von einem Alkohol hergestellt wird, ausgewählt aus den Verbindungen der Formel (III), 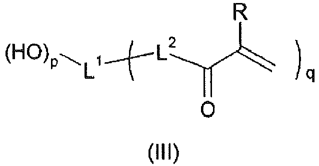 wobei:
▪ p für 1, 2, 3, 4 oder 5 steht;
▪ L¹ für einen linearen oder verzweigten Kohlenwasserstoffrest oder einen linearen oder verzweigten Rest steht, der eine Kohlenwasserstoffkette und mindestens ein Heteroatom umfasst;
▪ L² für O, S oder eine Gruppe der Formel NT steht, wobei T für H oder eine lineare oder verzweigte C₁-C₈-Alkylgruppe steht und N für ein Stickstoffatom steht;
▪ R identisch oder verschieden für H oder eine lineare oder verzweigte C₁-C₈-Alkylgruppe steht;
▪ q für 1, 2, 3, 4 oder 5 steht.

13. Modifiziertes Allophanat nach Anspruch 1 bis 12, wobei die Reaktion (b) mit mindestens einem Ester durchgeführt wird, ausgewählt aus 2-Hydoxyalkyl(meth)acrylaten, 2-Hydroxyethyl(meth)acrylat, 2-Hydroxypropyl(meth)acrylat, 3-Hydroxypropyl(meth)acrylat, 4-Hydroxybutyl(meth)acrylat, 3-Hydroxy-2,2-dimethylpropyl(meth)acrylat, 2-Hydroxyethylacrylat, 2-Hydroxypropylacrylat, einem durch Veresterung mit Hydroxyalkyl(meth)acrylaten modifiziertem Caprolacton, einem durch Veresterung mit Hydroxyalkyl(meth)acrylaten modifiziertem ε-Caprolacton, einem durch Veresterung mit Hydroxyalkylacrylaten modifiziertes ε-Caprolacton, einem durch Veresterung mit 2-Hydroxyalkyl(meth)acrylaten modifiziertes ε-Caprolacton, einem durch Veresterung mit 2-Hydroxyalkylacrylaten modifiziertem ε-Caprolacton, Caprolactontriacrylat, Glycerindi(meth)acrylat, Trimethylolpropandi(meth)acrylat, Pentaerythrittri(meth)acrylat (PETIA), Di(pentaerythrit)penta(meth)acrylat oder Trimethylolpropandiacrylat.

14. Modifiziertes Allophanat nach Anspruch 1 bis 13, wobei die Reaktion (b) mit mindestens einem Ester durchgeführt wird, ausgewählt aus Pentaerythrittriacrylat (PETIA), Dipentaerythritpentaacrylat (DPHA) oder Trimethylolpropandiacrylat.

15. Nutzung eines modifizierten Allophanats nach Anspruch 1 bis 14 zur Herstellung einer Beschichtungszusammensetzung, zur Herstellung einer vernetzbaren Beschichtungszusammensetzung, zur Herstellung einer durch UV-Strahlung vernetzbaren Beschichtungszusammensetzung, vorzugsweise zur Herstellung einer hydrophoben Beschichtungszusammensetzung, zur Herstellung einer hydrophoben vernetzbaren Beschichtungszusammensetzung oder einer durch UV-Strahlung vernetzbaren hydrophoben Beschichtungszusammensetzung.

## Claims

1. Modified allophanate that is or may be prepared according to a method comprising
(a) preparing an allophanate of formula (I) 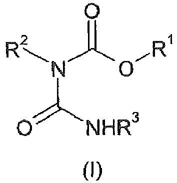 wherein
∘ R¹ is a monoalcohol compound that comprises an ether or polyether function after reaction of the hydrogen of the OH function of the monoalcohol with a compound having an isocyanate function;
∘ R² and R³, which are either identical or different, are a hydrocarbon group, comprising a derivative or non-derivative isocyanate function;
then
(b) reacting with at least one ester that
∘ is hydroxy-functionalised;
∘ comprises at least one acrylate function and
∘ is or may be prepared by reacting an acid, selected from acrylic acid and methacrylic acid, with at least one alcohol.

2. Modified allophanate according to claim 1, wherein the molar ratio of the urethane/allophanate functions is equal to 2.

3. Modified allophanate according to claims 1 or 2, wherein the allophanate of formula (I) has an NCO functionality selected from an NCO functionality ranging from 1.9 to 2.3; an NCO functionality ranging from 1.9 to 2.2; an NCO functionality ranging from 1.9 to 2.1; an NCO functionality ranging from 2 to 2.3; an NCO functionality ranging from 2 to 2.2; or
▪ wherein R¹ is a monoalcohol compound that does not comprise an acrylate function, R¹ preferably being an alcohol of formula R^{a}-[O-CH(R^{b})-CH₂]ₜ-OH, where R^{a} is a linear or branched C₁-C₂₀ alkyl group or a group of formula R^{c}-CO-, where R^{c} is a linear or branched C₁-C₂₀ alkyl group; R^{b} is H or an alkyl group, or a polyether group; t is an integer, t preferably being an integer between 1 and 10 or R^{b} being a C₁-C₈ alkyl group or a polyether group, or t being an integer from 1 to 5 or R^{b} being a methyl or a polyether group of formula -CH₂OR^{d}, where R^{d} is a hydrocarbon chain, or R^{d} is a polyoxyalkylene chain, a polyoxyethylene chain; or
▪ wherein R² and R³, which are identical or different, are a group comprising a derivative or non-derivative isocyanate function selected from an aliphatic, cycloaliphatic, heterocyclic or aromatic hydrocarbon group, R² and R³, which are identical or different, preferably being a group comprising a derivative or non-derivative isocyanate function selected from an aliphatic hydrocarbon group comprising a derivative or non-derivative isocyanate function.

4. Modified allophanate according to claims 1 to 3, wherein the allophanate of formula (I) is a homoallophanate, R² and R³ being identical, or wherein the allophanate of formula (I) is a mixed allophanate, R² and R³ being different.

5. Modified allophanate according to claims 1 to 4, comprising preparing an allophanate of formula (I) selected from a bis-allophanate or a tris-allophanate, preparing a mixture of other allophanates selected from one or more heavy allophanates, and preparing, in a minority manner, the isocyanate carbamate R²NCO and alcohol R¹OH, or the isocyanate carbamate R³NCO and alcohol R¹OH, or the isocyanate carbamates R²NCO and R³NCO and alcohol R¹OH.

6. Modified allophanate according to claims 1 to 5, wherein the allophanate of formula (I) is prepared from hexamethylene diisocyanate (HDI) or isophorone diisocyanate (IPDI).

7. Modified allophanate according to claims 1 to 6, comprising preparing (a) an allophanate of formula (I) and preparing at least one polyfunctional isocyanate.

8. Modified allophanate according to claims 1 to 7, comprising preparing (a) an allophanate of formula (I) and preparing at least one tricondensate polyfunctional isocyanate.

9. Modified allophanate according to claims 1 to 8, comprising preparing (a) an allophanate of formula (I) and preparing at least one polyfunctional isocyanate, in particular at least one tricondensate polyfunctional isocyanate, in a proportion of less than 10 wt.% based on the allophanate of formula (I); in a proportion of less than 8 wt.% based on the allophanate of formula (I); in a proportion of less than 6 wt.% based on the allophanate of formula (I); in a proportion of less than 2 wt.% based on the allophanate of formula (I).

10. Modified allophanate according to claims 1 to 9, wherein the reaction (b) is carried out using at least one monohydroxy-functionalised ester.

11. Modified allophanate according to claims 1 to 10, wherein the reaction (b) is carried out using just one ester or using two esters; or
wherein the reaction (b) is carried out using at least one alcohol not comprising an oxyalkylene or (poly)oxyalkylene group.

12. Modified allophanate according to claims 1 to 11, wherein the reaction (b) uses an ester prepared from an alcohol selected from the compounds of formula (III) 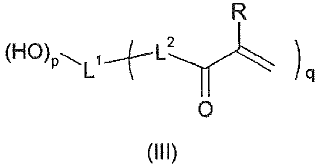 wherein
▪ p is 1, 2, 3, 4 or 5;
▪ L¹ is a linear or branched hydrocarbon radical, or a linear or branched radical, comprising a hydrocarbon chain and at least one heteroatom;
▪ L² is O, S or a group of formula NT, where T is H or a linear or branched C₁-C₈ alkyl group and N is a nitrogen atom;
▪ R, which is identical or different, is H or a linear or branched C₁-C₈ alkyl group;
▪ q is 1, 2, 3, 4 or 5.

13. Modified allophanate according to claims 1 to 12, wherein the reaction (b) is carried out using at least one ester selected from 2-hydroxyalkyl(meth)acrylates, 2-hydroxyethyl(meth)acrylate, 2-hydroxypropyl(meth)acrylate, 3-hydroxypropyl(meth)acrylate, 4-hydroxybutyl(meth)acrylate, 3-hydroxy-2,2-dimethylpropyl(meth)acrylate, 2-hydroxyethyl acrylate, 2-hydroxypropyl acrylate, a caprolactone modified by esterification using hydroxyalkyl(meth)acrylates, a ε-caprolactone modified by esterification using hydroxyalkyl(meth)acrylates, a ε-caprolactone modified by esterification using hydroxyalkylacrylates, a ε-caprolactone modified by esterification using 2-hydroxyalkyl(meth)acrylates, a ε-caprolactone modified by esterification using 2-hydroxyalkylacrylates, caprolactone triacrylate, glycerol di(meth)acrylate, trimethylolpropane di(meth)acrylate, pentaerythritol tri(meth)acrylate (PETIA), dipentaerythritol penta(meth)acrylate, trimethylolpropane diacrylate.

14. Modified allophanate according to claims 1 to 13, wherein the reaction (b) is carried out using at least one ester selected from pentaerythritol triacrylate (PETIA), dipentaerythritol pentaacrylate (DPHA) and trimethylolpropane diacrylate.

15. Use of a modified allophanate according to claims 1 to 14 for preparing a coating composition, preparing a cross-linkable coating composition or preparing a coating composition that is cross-linkable by UV radiation, preferably for preparing a hydrophobic coating composition, preparing a cross-linkable hydrophobic coating composition or preparing a hydrophobic coating composition that is cross-linkable by UV radiation.
